# EUROPEAN PATENT APPLICATION

(11) **EP 4 375 380 A1**
(43) Date of publication of application: **29.05.2024**
(21) Application number: 22845467.4
(22) Date of filing: 22.07.2022
(51) Int. Cl.: C12Q 1/68, C07K 14/185, A61K 39/395

(54) **DRUG FOR PREVENTING, ALLEVIATING OR TREATING MUCOSAL ADHESION AND USE THEREOF**

(30) Priority: 22.07.2021 CN 202110833123
(71) Applicant: SHANGHAI SYNVIDA BIOTECHNOLOGY CO. LTD., Shanghai 201303 (CN)
(72) Inventor: LIU, Junling, Shanghai 201203 (CN); FAN, Xuemei, Shanghai 201203 (CN); LIU, Yanjun, Shanghai 201203 (CN)
(74) Representative: Delbarba, Andrea
(86) International application number: PCT/CN2022/107526
(87) International publication number: WO 2023/001304

(57) **Abstract**

Provided is a drug for preventing, alleviating or treating adhesion and a use thereof. A fibroblast which has been subjected to external stimulation in a body cavity contains an activated negative regulatory receptor PEAR1. The deletion of the fibroblast PEAR1 can significantly promote in vivo mucosal adhesion formation. A PEARI agonist can specifically inhibit fibroblast activation, synthesize an extracellular matrix, and ameliorate the adhesion lesion. By administering the PEARI agonist, the formation of adhesion can be effectively prevented. Also provided is a PEARI agonistic antibody that does not affect the repair and healing of normal tissue and does not cause the tissue to be in an anoxic state. Moreover, the antibody drug has a long half-life, is easily diffused, has few side effects, and has a convenient route of administration.

## Description

This application claims priority to Chinese application No. 202110833123.3 filed on July 22, 2021.

### Technical Field

The disclosure belongs to the field of biomedicine and relates to a target for alleviating or treating adhesion and regulatory molecules thereof, specifically relates to a use of Pearl as a target in alleviating or treating adhesion, and further relates to regulatory molecules (such as antibodies) targeting Pearl and uses thereof.

### Technical Background

In human and animal, the mucosa is an important part of the body and is widely distributed, mainly covering the digestive system, urinary system, respiratory system, oral cavity, joints, inner layer of the skin, etc. The mucosa constitutes a barrier for the immune system of the body, resisting the invasion of harmful substances or pathogenic microorganisms. However, it can also be damaged, leading to mucosal lesions. The mucosa contains blood vessels and nerves and can secrete mucus.

Various factors UI can lead to mucosal adhesion, such as mechanical injury, infection, etc. For example, although surgical procedures can achieve therapeutic effects, they can cause varying degrees of damage to tissues such as the peritoneum, leading to the formation of fibrous adhesions, including adhesion-related encapsulating peritonitis, intra-abdominal adhesions, pelvic adhesions, pericardial adhesions, epidural adhesions, periodontal adhesions, and joint cavity adhesions. These conditions pose a significant threat to the patient's recovery and prognosis. For instance, intra-abdominal adhesions can cause complications such as intestinal adhesions and bowel obstruction, leading to abdominal pain and potentially lifethreatening complications such as intestinal necrosis and female infertility. Additionally, adhesion is the main reason of difficult tissue separation during secondary surgery with increased risk of bleeding.

Mucosal adhesion usually extends from one tissue to another, occurring on the surfaces of two injured tissues, where extracellular matrix proteins are deposited on the damaged tissue, leading to tissue adhesion. The mechanism of surgical adhesion formation is complex and it is a result of repairing damaged tissues. It is associated with various factors such as the sharp, mechanical, or thermal injuries caused by surgery, infection, heat radiation, local ischemia, dehydration, foreign body and so on. A series of reactions occur on the basis of tissue trauma, leading to the release of large amounts of vasoactive substances such as histamine and kinins, the increase of local vascular permeability, the damage of oxidative stress on the basis of hypoxia in local tissues and the release of large amounts of free oxygen and nitrogen radicals locally, further inducing local inflammatory reactions and exacerbating tissue damages. Subsequently, extracellular matrix proteins are deposited locally, containing a large number of infiltrated white blood cells and macrophages. Later, the healing process is completed through the activation of matrix protein deposition and fibroblast proliferation. The deposition of extracellular fibronectin predominates, resulting in early adhesions. After that, fibroblasts invade along with blood vessels, local vascularization occurs, and permanent adhesions are formed.

Although there are also some anti-adhesion drugs in this field, such as anti-adhesion films and anti-adhesion gels that can be implanted into wounds before the end of surgery to prevent postoperative adhesions, current anti-adhesion films and other drugs are all isolated physiologically, causing hypoxia in tissues sometimes and affecting wound healing. Therefore, there is an urgent need in this field to develop new anti-adhesion drugs that do not affect tissue healing and prevent tissue and organ adhesions.

### Summary of the Invention

The purpose of the present disclosure is to provide a drug for alleviating or treating mucosal adhesions and a use thereof.

In the first aspect, the present disclosure provides a use of Pearl (Platelet Endothelial Aggregation Receptor1) gene, an encoded protein or an agonist thereof in the manufacture of pharmaceutical compositions for preventing, alleviating or treating adhesive diseases.

In an embodiment, the adhesive diseases comprise: mucosal adhesive diseases or serosal adhesive diseases. preferably, the adhesive diseases comprise (but are not limited to): adhesive diseases formed after injuries, bleeding, infections, foreign body stimuli, tumor infiltration, or inflammatory cell infiltration; preferably, the adhesive diseases formed after injuries comprise postoperative adhesions, adhesions after radiotherapy or chemotherapy; the adhesive diseases formed after infections comprise inflammatory adhesions. Preferably, the adhesive diseases comprise (but are not limited to): adhesive diseases in digestive system, urinary system, respiratory system, reproductive system, oral cavity, joints and the inner layers of the skin; more preferably comprising adhesive diseases in abdominal cavity, pelvic cavity, thoracic cavity, uterus, joint cavities, pericardium, epidural space or periodontal sites.

In another embodiment, the adhesive diseases are abdominal adhesions, pelvic adhesions, thoracic adhesions or joint cavity adhesions.

In another embodiment, the agonist of Pearl comprises agents that increase the expression, activity, stability, and/or effective time of Pearl; preferably, it comprises (but are not limited to): an antibody that specifically activates Pearl, an antigen-binding fragment or a variant thereof, an expression construct for recombinantly expressing Pearl, a chemical agonist of Pearl, an up-regulator that promotes the driving ability by a promoter of Pearl gene; more preferably, the chemical agonist of Pearl comprise: FcεR1α, dextran sulfate, fucoidan, or combinations thereof.

In another embodiment, the Pearl gene, encoded protein or agonist thereof is also used for inhibiting mucosal fibroblast activation; reducing the content of hydroxyproline in tissues; reducing the thickness of the mucosa at the lesion site; inhibiting fibroblast (for example, myofibroblasts) proliferation or activation at the lesion site; decreasing the synthesis of collagen at the lesion site; or down-regulating adhesion-related indicators, preferably the indicators comprise: fibrinogen (FIB), α-smooth actin (α-SMA), t-PA/PAI, MMPs/TIMPs, transforming growth factor (TGF-β1), vascular endothelial growth factor (VEGF), tumor necrosis factor (TNF-α) and/or interleukin (IL-6, IL-17).

In another aspect, the present disclosure provides a method of preventing, alleviating or treating adhesive diseases, comprising administering the Pearl gene, encoded protein or agonist thereof to a subject in need of prevention, alleviation or treatment.

In another aspect, the present disclosure provides a pharmaceutical composition or drug kit for preventing, alleviating or treating adhesive diseases, wherein it comprises an effective amount of Pearl gene, encoded protein or agonist thereof, and a pharmaceutically acceptable carrier.

In another embodiment, based on physical form, the formulation of pharmaceutical composition comprises a formulation selected from liquid formulations such as syrups, injections, suspensions; solid formulations such as lyophilized agents, powders, granules, pills; semi-solid formulations such as ointments, pastes; gas formulations such as aerosols, dry powder inhalers, aerosol inhalers.

In another embodiment, based on administration routes, the formulation of pharmaceutical composition comprises a formulation selected from injectable formulations (such as Intracavitary (non-venous), intravenous, intramuscular, subcutaneous, intradermal, and acupoint injections), transdermal formulations (such as lotions, coatings, ointments, transdermal plasters, etc.), mucosal formulations (such as oral films, sublingual tablets, mouthwashes), gastrointestinal formulations (such as decoctions, mixtures, granules, pills, tablets, etc.), inhalation formulations for respiratory delivery (such as aerosols, dry powder inhalers, aerosol inhalers), rectal formulations (such as suppositories, enemas), or combinations thereof.

In another embodiment, in the pharmaceutical composition or drug kit, the component or composition can be administered by local coating, local injection, transdermal absorption, subcutaneous injection, inhalation, pulmonary nebulization.

In another embodiment, the pharmaceutical composition further comprises one or more of FcεR1α, dextran sulfate, fucoidan, aptamers and small molecule compounds as Pearl agonists; more preferably comprising FcεR1α, dextran sulfate and fucoidan.

In another embodiment, the agonist is an antibody that specifically activates Pearl, an antigen-binding fragment or variant thereof; preferably, the antibody comprises a heavy chain variable region and/or a light chain variable region, and the HCDR1, HCDR2 and HCDR3 amino acid sequences of the heavy chain variable region are as shown in SEQ ID NO: 26, 27 and 28, or as shown in SEQ ID NO: 26, 27 and 29, or as shown in SEQ ID NO: 26, 30 and 28, or as shown in SEQ ID NO: 31, 27 and 32, or as shown in SEQ ID NO: 33, 34 and 35; and/or the LCDR1, LCDR2 and LCDR3 amino acid sequences of the light chain variable region are as shown in SEQ ID NO: 45, GAT and SEQ ID NO: 46, or as shown in SEQ ID NO: 47, SAS and SEQ ID NO: 28, or as shown in SEQ ID NO: 49, DTS and SEQ ID NO: 50, or as shown in SEQ ID NO: 51, GAT and SEQ ID NO: 52.

In another embodiment, the heavy chain variable region of the antibody is as shown in SEQ ID NO: 1, 2, 3, 4, 5, 7, 8, 9, 10, 11, 12, 13, 14 or 15, or the light chain variable region of the antibody is as shown in SEQ ID NO: 16, 17, 18, 19, 20, 22, 23, 24 or 25.

In another embodiment, the antibody comprises a heavy chain variable region and/or a light chain variable region, and the HCDR1, HCDR2 and HCDR3 amino acid sequences of the heavy chain variable region are as shown in SEQ ID NO: 26, SEQ ID NO: 27 and SEQ ID NO: 28; the LCDR1, LCDR2 and LCDR3 amino acid sequences of the light chain variable region are as shown in SEQ ID NO: 45, GAT and SEQ ID NO: 46, or the LCDR1, LCDR2 and LCDR3 amino acid sequences of the light chain variable region are as shown in SEQ ID NO: 47, GTS and SEQ ID NO: 48, or the LCDR1, LCDR2 and LCDR3 amino acid sequences of the light chain variable region are as shown in SEQ ID NO: 49, DTS and SEQ ID NO: 50, or the LCDR1, LCDR2 and LCDR3 amino acid sequences of the light chain variable region are as shown in SEQ ID NO: 51, GAT and SEQ ID NO: 52, or the LCDR1, LCDR2 and LCDR3 amino acid sequences of the light chain variable region are as shown in SEQ ID NO: 53, LVS and SEQ ID NO: 54.

In another embodiment, the variant of the antibody has a sequence with an addition, deletion or substitution of one or several, preferably within 1, 2, 3, 4, 5, 6, 7, 8, 9, or 10 amino acid residues in the CDR, FR or full-length sequence.

In another embodiment, the variant has or substantially has the activity of targeting and activating Pearl.

In another embodiment, the HCDR1 of the variant has a mutation from S to A or T at the third position, a mutation from G to D at the sixth position, and/or, a mutation from P to T or Y at the eighth position in the amino acid sequence shown in SEQ ID NO: 26; and/or, the HCDR2 of the variant has a mutation from P to S or G at the third position, a mutation from Y to N at the fourth position, and/or, a mutation from T to A at the eighth position in the amino acid sequence shown in SEQ ID NO:27; and/or, the HCDR3 of the variant has a mutation from A to D at the ninth position and/or, a mutation from Y to F at the tenth position in the amino acid sequence shown in SEQ ID NO:28.

In another embodiment, in the antibody or the variant thereof, HCDR1 comprises any one of the amino acid sequences selected from SEQ ID NO: 26, 31, 33, 36 and SEQ ID NO: 39; HCDR2 comprises any one of the amino acid sequences selected from SEQ ID NO: 27, 30, 34, 37, and SEQ ID NO: 40-44; and, HCDR3 comprises any one of the amino acid sequences selected from SEQ ID NO: 28, 29, 32, 35 and SEQ ID NO: 38; and/or, the LCDR1 comprises any one of the amino acid sequences selected from SEQ ID NO: 45, 47, 49, 51 and SEQ ID NO: 53; LCDR2 comprises any one of the amino acid sequences selected from GAT, SAS, DTS, and LVS; and, LCDR3 comprises any one of the amino acid sequences selected from SEQ ID NO: 46, 48, 50, 52 or SEQ ID NO: 54.

In another embodiment, in the antibody or the variant thereof, the HFR (heavy chain variable region framework region) 1 comprises any one of the amino acid sequences selected from SEQ ID NO: 55, 56 and SEQ ID NO: 57; HFR2 comprises any one of the amino acid sequences selected from SEQ ID NO: 58, 59, 60, 61, 62, 63, 64, 65 and SEQ ID NO: 66; and, HFR3 comprises any one of the amino acid sequences selected from SEQ ID NO: 67 , 68, 69, 70, 71, 72, 73, 74, 75 and SEQ ID NO: 76; and HFR4 comprises any one of the amino acid sequences selected from SEQ ID NO: 77 and SEQ ID NO: 78.

In another embodiment, in the antibody or the variant thereof, the LFR (framework region of light chain variable region) 1 comprises any one of the amino acid sequences selected from SEQ ID NO: 79, 80, 81, 82, 83 and SEQ ID NO: 84; LFR2 comprises any one of the amino acid sequences selected from SEQ ID NO: 85, 86, 87, 88, 89 and SEQ ID NO: 90; and LFR3 comprises any one of the amino acid sequences selected from SEQ ID NO: 91, 92, 93, 94, 95, 96, 97 and SEQ ID NO: 98; and LFR4 comprises any one of the amino acid sequences selected from SEQ ID NO: 99, 100, 101, 102 and SEQ ID NO: 103.

In another embodiment, the antibody or the variant thereof comprises an amino acid sequences respectively:
HCDR1, HCDR2 and HCDR3 as shown in SEQ ID NO: 26, SEQ ID NO: 27, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 26, SEQ ID NO: 27, SEQ ID NO: 29, LCDR1, LCDR2 and LCDR3 as shown in SEQ ID NO: 45, GAT, SEQ ID NO: 46; or,
HCDR1, HCDR2 and HCDR3 as shown in SEQ ID NO: 26, SEQ ID NO: 30, SEQ ID NO: 28, LCDR1, LCDR2 and LCDR3 as shown in SEQ ID NO: 47, GTS, SEQ ID NO: 48; or,
HCDR1, HCDR2 and HCDR3 as shown in SEQ ID NO: 26, SEQ ID NO: 27, SEQ ID NO: 29, LCDR1, LCDR2 and LCDR3 as shown in SEQ ID NO: 49, DTS, SEQ ID NO: 50; or,
HCDR1, HCDR2 and HCDR3 as shown in SEQ ID NO: 26, SEQ ID NO: 40, SEQ ID NO: 28, LCDR1, LCDR2 and LCDR3 as shown in SEQ ID NO: 49, GAT, SEQ ID NO: 54; or,
HCDR1, HCDR2 and HCDR3 as shown in SEQ ID NO: 26, SEQ ID NO: 41, SEQ ID NO: 28, LCDR1, LCDR2 and LCDR3 as shown in SEQ ID NO: 49, SAS, SEQ ID NO: 54; or,
HCDR1, HCDR2 and HCDR3 as shown in SEQ ID NO: 26, SEQ ID NO: 42, SEQ ID NO: 28, LCDR1, LCDR2 and LCDR3 as shown in SEQ ID NO: 49, LVS, SEQ ID NO: 52; or,
HCDR1, HCDR2 and HCDR3 as shown in SEQ ID NO: 26, SEQ ID NO: 43, SEQ ID NO: 32, LCDR1, LCDR2 and LCDR3 as shown in SEQ ID NO: 45, GAT, SEQ ID NO: 54; or,
HCDR1, HCDR2 and HCDR3 as shown in SEQ ID NO: 31, SEQ ID NO: 27, SEQ ID NO: 32, LCDR1, LCDR2 and LCDR3 as shown in SEQ ID NO: 51, GAT, SEQ ID NO: 52; or,
HCDR1, HCDR2 and HCDR3 as shown in SEQ ID NO: 31, SEQ ID NO: 37, SEQ ID NO: 32, LCDR1, LCDR2 and LCDR3 as shown in SEQ ID NO: 53, LVS, SEQ ID NO: 46; or,
HCDR1, HCDR2 and HCDR3 as shown in SEQ ID NO: 31, SEQ ID NO: 34, SEQ ID NO: 32, LCDR1, LCDR2 and LCDR3 as shown in SEQ ID NO: 45, SAS, SEQ ID NO: 50; or,
HCDR1, HCDR2 and HCDR3 as shown in SEQ ID NO: 31, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 31, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 33, SEQ ID NO: 34, SEQ ID NO: 35, LCDR1, LCDR2 and LCDR3 as shown in SEQ ID NO: 45, GAT, SEQ ID NO: 46; or,
HCDR1, HCDR2 and HCDR3 as shown in SEQ ID NO: 33, SEQ ID NO: 37, SEQ ID NO: 32, LCDR1, LCDR2 and LCDR3 as shown in SEQ ID NO: 53, LVS, SEQ ID NO: 46; or,
HCDR1, HCDR2 and HCDR3 as shown in SEQ ID NO: 33, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 33, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 36, SEQ ID NO: 37, SEQ ID NO: 38, LCDR1, LCDR2 and LCDR3 as shown in SEQ ID NO: 53, LVS, SEQ ID NO: 54; or,
HCDR1, HCDR2 and HCDR3 as shown in SEQ ID NO: 36, SEQ ID NO: 34, SEQ ID NO: 32, LCDR1, LCDR2 and LCDR3 as shown in SEQ ID NO: 45, SAS, SEQ ID NO: 50; or,
HCDR1, HCDR2 and HCDR3 as shown in SEQ ID NO: 36, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 36, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 39, SEQ ID NO: 27, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 32, LCDR1, LCDR2 and LCDR3 as shown in SEQ ID NO: 45, SAS, SEQ ID NO: 50; or,
HCDR1, HCDR2 and HCDR3 as shown in SEQ ID NO: 39, SEQ ID NO: 30, SEQ ID NO: 35, LCDR1, LCDR2 and LCDR3 as shown in SEQ ID NO: 47, GAT, SEQ ID NO: 50; or,
HCDR1, HCDR2 and HCDR3 as shown in SEQ ID NO: 39, SEQ ID NO: 42, SEQ ID NO: 28, LCDR1, LCDR2 and LCDR3 as shown in SEQ ID NO: 49, DTS, SEQ ID NO: 54; or,
HCDR1, HCDR2 and HCDR3 as shown in SEQ ID NO: 39, SEQ ID NO: 37, SEQ ID NO: 28, LCDR1, LCDR2 and LCDR3 as shown in SEQ ID NO: 49, GAT, SEQ ID NO: 48; or,
HCDR1, HCDR2 and HCDR3 as shown in SEQ ID NO: 39, SEQ ID NO: 40, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 41, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 42, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 43, SEQ ID NO: 28, LCDR1, LCDR2 and LCDR3 as shown in SEQ ID NO: 45, GAT, SEQ ID NO: 46; or,
HCDR1, HCDR2 and HCDR3 as shown in SEQ ID NO: 39, SEQ ID NO: 44, SEQ ID NO: 28, LCDR1, LCDR2 and LCDR3 as shown in SEQ ID NO: 45, GAT, SEQ ID NO: 46.

In another embodiment, the amino acid sequences of the light chain variable region and the heavy chain variable region are respectively shown in SEQ ID NO: 16 and SEQ ID NO: 1; or, the amino acid sequences of the light chain variable region and the heavy chain variable region are respectively are shown in SEQ ID NO: 16 and SEQ ID NO: 2; or, the amino acid sequences of the light chain variable regions and the heavy chain variable regions are respectively shown in SEQ ID NO: 17 and SEQ ID NO: 3; or, the amino acid sequences of the light chain variable region and the heavy chain variable region are respectively shown in SEQ ID NO: 18 and SEQ ID NO: 2; or, the amino acid sequences of the light chain variable region and the heavy chain variable region are respectively shown in SEQ ID NO: 19 and SEQ ID NO: 4; or, the amino acid sequences of the light chain variable region and the heavy chain variable region are respectively shown in SEQ ID NO: 20 and SEQ ID NO: 5; or, the amino acid sequences of the light chain variable region and the heavy chain variable region are respectively shown in SEQ ID NO: 21 and SEQ ID NO: 6; or, the amino acid sequences of the light chain variable region and the heavy chain variable region are respectively shown in SEQ ID NO: 22 and SEQ ID NO: 7; or, the amino acid sequences of the light chain variable region and the heavy chain variable region are respectively shown in SEQ ID NO : 23 and SEQ ID NO: 7; or, the amino acid sequences of the light chain variable region and the heavy chain variable region are respectively shown in SEQ ID NO: 24 and SEQ ID NO: 7; or, the amino acid sequences of the light chain variable region and the heavy chain variable region are respectively shown in SEQ ID NO: 25 and SEQ ID NO: 7; or, the amino acid sequences of the light chain variable region and the heavy chain variable region are respectively shown in SEQ ID NO: ID NO: 22 and SEQ ID NO: 8; or, the amino acid sequences of the light chain variable region and the heavy chain variable region are respectively shown in SEQ ID NO: 23 and SEQ ID NO: 8; or, the amino acid sequences of the light chain variable region and the heavy chain variable region are respectively shown in SEQ ID NO: 24 and SEQ ID NO: 8; or, the amino acid sequences of the light chain variable region and the heavy chain variable region are respectively shown in SEQ ID NO: 25 and SEQ ID NO: 8; or, the amino acid sequences of the light chain variable region and the heavy chain variable region are respectively shown in SEQ ID NO: 22 and SEQ ID NO: 9; or, the amino acid sequences of the light chain variable region and the heavy chain variable region are respectively shown in SEQ ID NO: 23 and SEQ ID NO: 9; or, the amino acid sequences of the light chain variable region and the heavy chain variable region are respectively shown in SEQ ID NO: 24 and SEQ ID NO: 9; or, the amino acid sequences of the light chain variable region and the heavy chain variable region are respectively shown in SEQ ID NO: 25 and SEQ ID NO: 9 or, the amino acid sequences of the light chain variable region and the heavy chain variable region are respectively shown in SEQ ID NO: 22 and SEQ ID NO: 10; or, the amino acid sequences of the light chain variable region and the heavy chain variable region are respectively shown in SEQ ID NO: 23 and SEQ ID NO: 10; or, the amino acid sequences of the light chain variable region and the heavy chain variable region are respectively shown in SEQ ID NO: 24 and SEQ ID NO: 10; or, the amino acid sequences of the light chain variable region and the heavy chain variable region are respectively shown in SEQ ID NO: 25 and SEQ ID NO: 10; or, the amino acid sequences of the light chain variable region and the heavy chain variable region are respectively shown in SEQ ID NO: 22 and SEQ ID NO: 11 or, the amino acid sequences of the light chain variable region and the heavy chain variable region are respectively shown in SEQ ID NO: 22 and SEQ ID NO: 12; or, the amino acid sequences of the light chain variable region and the heavy chain variable region are respectively shown in SEQ ID NO: 22 and SEQ ID NO: 13 respectively; or, the amino acid sequences of the light chain variable region and the heavy chain variable region are respectively shown in SEQ ID NO: 22 and SEQ ID NO: 14; or, the amino acid sequences of the light chain variable region and the heavy chain variable region are respectively shown in SEQ ID NO: 22 and SEQ ID NO: 15.

In another embodiment, the variant is 80%, 85%, 90%, 95%, 96%, 97%, 98%, or 99% identical to the amino acid sequence of the antibody.

In another embodiment, the antibody is a full-length antibody, Fab, Fab', F(ab')₂, Fv such as scFv, bispecific antibody, multispecific antibody, or single domain antibody, or the monoclonal or polyclonal antibodies produced by the above-mentioned antibody.

In another embodiment, the antibody or variant thereof is a full-length antibody, and the heavy chain constant region of the full-length antibody is selected from heavy chain constant regions of hIgG1, hIgG2, hIgG3 or hIgG4 or a derivative sequence thereof.

In another embodiment, the light chain constant region of the antibody is selected from κ chain or λ chain or a derivative sequence thereof.

In another embodiment, the derivative sequence of the heavy chain constant region comprises a sequence of heavy chain constant region of hIgG1, hIgG2, hIgG3 or hIgG4 with an addition, deletion or substitution of one or several, preferably within 1, 2, 3, 4, 5, 6, 7, 8, 9, or 10 amino acid residues.

In another embodiment, the derivative sequence of the heavy chain constant region is 80%, 85%, 90%, 95%, 96%, 97%, 98%, or 99% identical to the amino acid sequence of the heavy chain constant region of hIgG1, hIgG2, hIgG3 or hIgG4.

In another embodiment, the heavy chain constant region of the full-length antibody is the heavy chain constant region of hIgG3 or hIgG4 or a derivative sequence thereof.

In another embodiment, the derivative sequence is an amino acid sequence after 1-2 amino acid substitutions at the heavy chain constant region of hIgG1, hIgG2, hIgG3 or hIgG4.

In another embodiment, the derivative sequence has a mutation of S228P in hIgG4.

In another embodiment, the light chain constant region is human κ chain.

In another embodiment, the expression construct (expression vectors) comprise: viral vectors, non-viral vectors; for example, the expression vectors comprise: adeno-associated virus vectors, lentiviral vectors, adenoviral vectors.

In another aspect, the present disclosure provides a use of Pearl gene, an encoded protein thereof for screening drugs or compounds (i.e. as drug screening targets) for preventing, alleviating or treating adhesive diseases.

In another aspect, the present disclosure provides a method of screening drugs or compounds (or potential drugs or compounds) for preventing, alleviating or treating adhesive diseases, comprising: (1) treating an expression system by a candidate substance, wherein the system expressing Pearl or comprising Pearl; and (2) detecting the system to observe the expression or activity of Pearl; if the expression or activity of Pearl is elevated statistically by the candidate substance, then it indicates that the candidate substance is the desired (interested) drug or compound.

In another embodiment, the step (1) comprises: in the testing group, adding candidate substances to the expression system; and/or, the step (2) comprises: detecting the system to observe the expression or activity of Pearl and compared to the control group, wherein the control group is an expression system without adding the candidate substances; if the expression or activity of Pearl is elevated statistically (such as elevated by more than 20%, preferably more than 50%; more preferably more than 80%) by the candidate substance, then it indicates that the candidate substance is the desired (interesting) drug or compound.

In another embodiment, the system is selected from: cell system (or cell culture system), subcellular system (or subcellular culture system), solution system, tissue system, organ system or animal system.

In another embodiment, the system is cell system or cell culture system. Preferably, the cell expresses or over-expresses Pearl; or the cell is treated by Pearl, wherein the content of Pearl is 0.001nM-1M.

In another embodiment, the cell comprises mucosal fibroblasts.

In another embodiment, when the screening is based on mucosal fibroblasts, it can be further judged/assistantly judged by detecting the ability of fibroblasts to synthesize extracellular matrix. If compared with the control group, the ability of cells in the testing group to synthesize extracellular matrix is weakened, then the corresponding candidate substance is a potentially desired (interesting) drug or compound.

In another embodiment, the system is an animal.

In another embodiment, the animal is a Pearl humanized transgenic animal.

In another embodiment, the animal comprises mice, rats, cats, dogs, monkeys, camels, alpacas, or combinations thereof.

In another embodiment, when the screening is based on the animal, it can be further judged/assistantly judged by BhatiaDS score or Zuhlke score. If compared with the control group, the BhatiaDS score or Zuhlke score decreased significantly in the testing group, then the corresponding candidate substance is a potentially desired (interesting) drug or compound.

In another embodiment, when the screening is based on the animal, it can be further judged/assistantly judged by measuring the hydroxyproline in tissues. If compared with the control group, the content of hydroxyproline in tissues decreased significantly in the testing group, then the corresponding candidate substance is a potentially desired (interesting) drug or compound.

In another embodiment, when the screening is based on the animal, it can be further judged/assistantly judged by determining adhesion-related indicators (such as fibrinogen (FIB), α-smooth actin (α-SMA), t-PA/PAI, MMPs/TIMPs, transforming growth factor (TGF-β1), vascular endothelial growth factor (VEGF), tumor necrosis factor (TNF-α) and/or interleukin (IL-6, IL-17)). If compared with the control group, the adhesion-related indicators decreased significantly in the testing group, then the corresponding candidate substance is a potentially desired (interesting) drug or compound.

In another embodiment, when the screening is based on the animal, it can be further judged/assistantly judged by measuring the thickness of subcutaneous mucosa and skin, the proliferation, overactivation and collagen synthesis of fibroblasts. If compared with the control group, the thickness of subcutaneous mucosa and skin, the proliferation, overactivation and collagen synthesis of fibroblasts decreased significantly in the testing group, then the corresponding candidate substance is a potentially desired (interesting) drug or compound.

In another embodiment, the activity is an activity of activating Pearl.

In another embodiment, the potential substance comprise (but are not limited to): antibodies (monoclonal antibodies or polyclonal antibodies) designed against Pearl, fragments or variants thereof, coding genes thereof, or upstream and downstream molecules thereof or signaling pathways), over-expressive/deleted/down-regulated molecules such as constructs, promoters or inhibitors, small chemical molecules, interacting molecules, etc.

In another embodiment, the method also comprises: further cell experiments and/or animal experiments on the obtained drug or compound (potential drug or compound), so as to further select and determine compositions useful for preventing, alleviating or treating adhesive diseases.

Other aspects of the present disclosure will be apparent to those skilled in the art based on the disclosure herein.

### Description of Figures

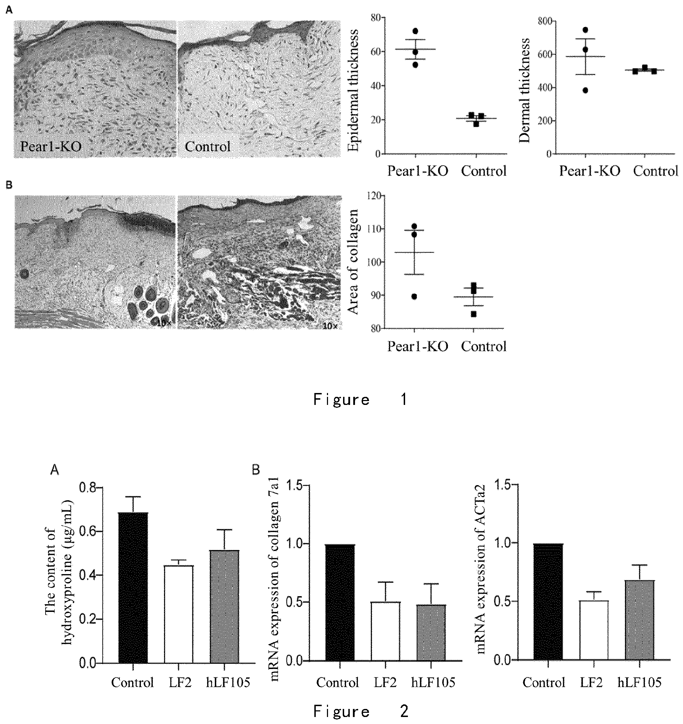

Figure 1. Pearl-deleted mice formed more severe subcutaneous mucosal adhesions than mice in the control group. A. HE staining at wounded sites showed that the fibroblasts of Pearl-deficient mice proliferated in large numbers at wounded sites, and the larger activated myofibroblasts increased. The thickness of epidermis and dermis was counted by Image J software. B. Masson staining at wounded sites was carried out, with the area of collagen being counted.
Figure 2. Anti-Pear1 monoclonal antibody inhibits the activation of mucosal fibroblasts. A. The content of hydroxyproline in cell supernatant was measured. B. Expression level of extracellular matrix protein collagen 1a2, smooth muscle actin α2 (ACTA2) in fibroblasts by QPCR detection.

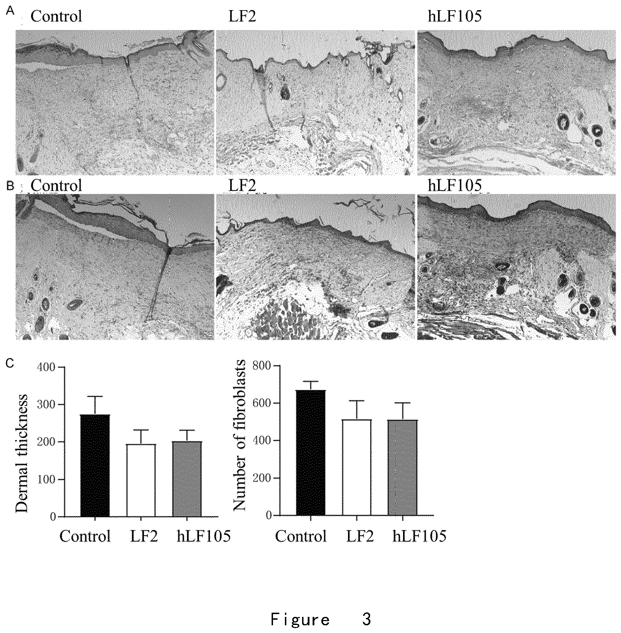

Figure 3. Anti-human Pearl monoclonal antibody can inhibit the formation of adhesions. A. HE staining at wounded sites; B. Masson staining at wounded sites showed that there is less collagen deposition in the subcutaneous mucosa at wounded sites in mice treated with anti-Pearl monoclonal antibody LF2 and hLF105 compared with the control group. C. Statistical analysis of HE staining. It can be seen that compared with the control group, there is fewer fibroblasts and smaller dermal thickness at wounded sites in mice treated with anti-Pearl monoclonal antibody LF2 and hLF105.

### Detailed Description

The inventors firstly found that a fibroblast which has been subjected to external stimulation in a body cavity contains an activated negative regulatory receptor PEAR1. The deletion of the fibroblast Pearl can significantly promote in vivo mucosal adhesion formation. A Pearl agonist can specifically inhibit fibroblast activation, synthesize an extracellular matrix, and ameliorate the adhesion lesion. By administering the Pearl agonist, the formation of adhesion can be effectively prevented. Also provided is a PEARI agonistic antibody that does not affect the repair and healing of normal tissue and does not cause the tissue to be in an anoxic state. Moreover, the antibody drug has a long half-life, is easily diffused, has few side effects, and has a convenient route of administration.

The inventor has previously applied for a patent, using Pearl as a target of fibrotic diseases, constructing an antibody that enhances the activity of Pearl, inhibiting the excessive activation of fibroblasts, so as to achieve the purpose of treating fibrotic diseases. These applications comprise: Chinese patent application 202010076729.2 with a filing date of 2020/1/23 and Chinese patent application 202011212639.8 with a filing date of 2020/11/3. The above-mentioned Chinese patent applications were incorporated in the present disclosure in its entirety. The present disclosure further proposes a new treatment scheme related to alleviating or treating mucosal adhesions using Pearl as a regulatory target.

### Terms

As used herein, the term "mucosal" is used interchangeably with "mucosa", which is a membrane-like structure composed of epithelial and connective tissues in organisms (in oral cavity, organs, stomach, intestines, urinary tract, etc.). In the present disclosure, it may also refer to the epithelium and connective tissue facing the cavity of hollow organs or joint spaces.

As used herein, the terms "adhesion" and "adhesive" are used interchangeably.

As used herein, the term "enhance", "promote", and "improve", "increase" are used interchangeably; preferably statistically significantly "increase", "promote", "improve" or "enhance", such as an increase of 2% or higher, 5% or higher, 10% or higher, 15% or higher, 20% or higher, 30% or higher, 50% or higher, 80% or higher, 100% or higher, 200% or higher, 500% or higher.

As used herein, the term "reduce", "decrease", "weaken" are used interchangeably; preferably statistically significantly "inhibit", "reduce", "decrease" or "weaken", such as an decrease of 2% or lower, 5% or lower, 10% or lower, 15% or lower, 20% or lower, 30% or lower, 50% or lower, 80% or lower, 100% or lower, 200% or lower, 500% or lower.

As used herein, the term "treatment (treatment and treating)" comprise the administration of one or more active ingredients to prevent or delay the onset of the symptoms or complications, and to prevent or delay the development of the disease, condition or disorder, and/or to eliminate or control the disease, condition or disorder and alleviate symptoms or complications associated with the disease, condition or disorder.. The term "treatment" encompasses both therapeutic, i.e. curative and/or palliative treatment and prophylactic, i.e. prophylactic treatment. Therapeutic treatment refers to the treatment of a patient who has developed one or more of the manifested acute or chronic disorders. Therapeutic treatment may be symptomatic to alleviate the symptoms of a particular indication, or etiological to reverse or partially reverse the symptoms of the indication or to arrest or slow the progression of the disease. Prophylactic treatment ("prophylaxis") refers to therapeutic treatments of a patient at risk of developing one or more symptoms before the clinical onset of the disease, with the aim of reducing the risks.

As used herein, the term "targeting and activating" means that molecules in the testing group may activate the target genes, proteins or activities thereof, thereby up-regulating their expressions or activities. Usually, "targeting and activating" can be measured by one or more of the methods of gene expression or protein expression, the level of activity, downstream substances that affect pathways or secretion, etc.

As used herein, the term "subject" and "individual" refers to living animals (e.g., mammals), including but not limited to humans, non-human primates, rodents, etc., which will be the recipients of a particular treatment. Typically, the terms "subject" and "patient" are used interchangeably.

As used herein, the term "comprise" means that various components can be used together in the mixture or composition of the present disclosure. Therefore, the term "mainly consist of......" and "consist of......" is included in the term "comprise". The term "mainly consist of......" and "consist of......" is included in the term "comprise".

As used herein, "pharmaceutically acceptable" ingredients refer to substances suitable for use in humans and/or mammals without undue adverse side effects (such as toxicity), ie. with reasonable benefit/risk ratio. The term "pharmaceutically acceptable carrier" refers to a carrier for the administration of a therapeutic agent, comprising various excipients and diluents.

As used herein, "effective amount" means the amount of pharmaceutically active component in the present disclosure that (i) treats or prevents the particular disease or condition, (ii) attenuates, improves or eliminates one or more symptoms of the particular disease or disorder, or (iii) prevents or delays the onset of one or more symptoms of the particular disease or disorder described herein. The terms "effective amount", "safe and effective amount" or "therapeutically effective amount" are used interchangeably herein. Those skilled in the art (such as experienced clinicians) can adjust the "safe and effective dose" according to the subject.

As used herein, the term "antibody or variant thereof' refers to a protein, antibody fragment or polypeptide sequence derived from an immunoglobulin molecule that specifically binds to an antigen. The antibody may be polyclonal or monoclonal, multi-stranded or singlestranded, or an intact immunoglobulin, and may be derived from natural sources or recombinant sources. In a specific embodiment, an "antibody or variant" of the disclosure is isolated and derived from recombinant sources.

As used herein, the term "antibody fragment" refers to at least a portion of the antibody that retains the ability to specifically interact with an antigen (e.g., by binding, steric hindrance, stabilization/destabilization, spatial distribution). Examples of antibody fragments include, but are not limited to, Fab, Fab', F(ab')2, Fv fragment, scFv antibody fragment, disulfide bondlinked FVS (sdFV), Fd fragment consisting of VH and CH1 domains, linear antibodies, single domain antibodies, multi-specific antibodies formed by antibody fragments (e.g., bivalent fragments of two Fab fragments linked by disulfide bonds in a hinge region) and isolated CDRs or other epitope binding fragments. The antigen-binding fragment can also be incorporated into a single-domain antibody, a maximum antibody, a micro antibody, a nano-antibody, an intracellular antibody, a bi-specific antibody and a chimeric antibody.

Scope: In the entire disclosure, various aspects of the disclosure may be present in the form of ranges. It should be understood that the description in the form of ranges is for convenience and brevity and should not be construed as limiting the scope of the disclosure. Accordingly, the description of the ranges should be considered to specifically disclose all possible sub-ranges and individual numerical values within that range. For example, a description of a range, such as from 1 to 6, should be considered to specifically disclose sub-ranges such as 1 to 3, 1 to 4, 1 to 5, 2 to 4, 2 to 6, 3 to 6, etc. as well as individual numerical values within that range, such as 1, 2, 2.7, 3, 4, 5, 5.3, and 6. As another example, a range, such as having an identity of 95-99%, includes a range of identity of 95%, 96%, 97%, 98% or 99% including sub-ranges such as an identity of 96-99%, 96-98%, 96-97%, 97-99%, 97-98% and 98-99%. This is applicable regardless of the width of the range.

**Adhesive diseases**In the present disclosure, "adhesion", "adhesive disease", and "adhesive lesion" can be used interchangeably, and refer to the mucosal or serous lesion of the body. There are many predisposing factors, including injury, bleeding, infection, foreign body stimulation or tumor infiltration that can form adhesions, for example, postoperative adhesions, adhesions after radiotherapy or chemotherapy, inflammatory adhesions, and so on. The mucosa in the present disclosure refers to a membrane-like structure composed of epithelial and connective tissues in organisms (in oral cavity, organs, stomach, intestines, urinary tract, etc.). In the present disclosure, it may also refer to abnormal proliferative tissues resulting from excessive activation of fibroblasts and abnormal collagen accumulation in the epithelium and connective tissues facing the cavity of hollow organs, between organs, within body cavities, organs, or joint spaces.

Adhesive lesions can occur in various parts of the body, including but not limited to digestive system, urinary system, respiratory system, reproductive system, oral cavity, joints, inner layers of the skin, and so on. The formation of adhesions has diverse causes, complex mechanisms and a similar process. Taking surgical adhesions as an example, adhesions is a result of repairing damaged tissues and it is associated with various factors such as the sharp, mechanical, or thermal injuries caused by surgery, infection, heat radiation, local ischemia, dehydration, foreign body and so on. A series of reactions occur on the basis of tissue trauma, leading to the release of large amounts of vasoactive substances such as histamine and kinins, the increase of local vascular permeability, the damage of oxidative stress on the basis of hypoxia in local tissues and the release of large amounts of free oxygen and nitrogen radicals locally, further inducing local inflammatory reactions and exacerbating tissue damages. Subsequently, extracellular matrix proteins are deposited locally, containing a large number of infiltrated white blood cells and macrophages. Later, the healing process is completed through the activation of matrix protein deposition and fibroblast proliferation. The deposition of extracellular fibronectin predominates, resulting in early adhesions. After that, fibroblasts invade along with blood vessels, local vascularization occurs, and permanent adhesions are formed.

The symptoms of patients with adhesions may vary depending on the degree and location of adhesions. For example, patients with abdominal or intestinal adhesions may experience slowed or stalled intestinal motility. Mild cases may have no obvious discomfort symptoms, or occasionally experience mild abdominal pain and bloating after eating. Severe cases may often be accompanied by abdominal pain, bloating, poor gas elimination, belching, and dry stools. Normal intestinal motility can expel food residues and gas. However, in patients with intestinal adhesions, the narrowed intestinal tube hinders the passage of intestinal contents. As result, gases and feces inside the intestine cannot be smoothly expelled. As the accumulation of material increases, the pressure within the intestinal lumen also tends to rise, leading to intestinal obstruction. In addition, due to the presence of adhesions, there is a greater likelihood of ischemia, hypoxia and necrosis in local tissues. The damage of oxidative stress occurred on the basis of hypoxia in local tissues and large amounts of free oxygen and nitrogen radicals released locally, further inducing local inflammatory reactions and exacerbating tissue damages. This results in difficult tissue separation during secondary surgery, increased tissue fragility, enhanced bleeding in adjacent connected tissues and increased complexity of surgery.

The adhesive diseases in the present disclosure comprise but are not limited to diseases occurred inside the cavity of hollow organs, between organs, in the epithelium and connective tissues of organs, especially local parts after surgery, radiotherapy and chemotherapy. For example, the local parts are inside cavities such as the abdominal cavity, chest cavity, pelvic cavity, joint cavity and so on.

### Pearl gene or protein thereof and a use of being a target in screening drugs thereof

The present disclosure firstly disclosed that the deletion of the fibroblast Pearl can significantly promote in vivo mucosal adhesion formation and a PearI agonist can specifically inhibit fibroblast activation, synthesize an extracellular matrix, and alleviate the adhesion lesion.

Pearl, Platelet Endothelial Aggregation Receptor 1 (Gene ID: 375033), is a 150 kDa type I transmembrane protein, and is a member of a Multiple Epidermal Growth Factor-Like domain Receptor family (Multiple Epidermal Growth Factor-Like domain Receptor family, MEGF) Protein family. Human Pearl contains 1037 amino acids (aa), wherein a 20 aa signal sequence, a 735 aa extracellular domain (ECD), a 21 aa transmembrane region and a 261 aa cytoplasmic region are contained. Pearls express in all mammals and have high similarity. For example, the similarity between human Pearl and mouse Pearl (Gene ID II: 73182) is 84%.

In the present disclosure, for fibroblast-specific Pearl knockout animals, sham operation was carried out by peritoneal adhesion model in mice. The results showed that BhatiaDS score or Zuhlke score of the mice treated with Pearl agonist was significantly lower than that of the control group. Hydroxyproline assays in tissues showed that the content of hydroxyproline in animal tissues treated with Pearl agonist was significantly lower than that of the control group.

Animals treated with Pearl agonists had significantly lower adhesion-related indicators than those in the control group. Fibroblast-specific Pearl knockout animals were used to observe the formation of subcutaneous mucosal adhesions. The results showed that Pearl inhibited the excessive activation of mucosal fibroblasts and the formation of subcutaneous mucosal adhesions.

The present inventors detected the expression levels of extracellular matrix proteins such as collagen 7a1 (Col 7a1) and smooth muscle actin α2 (ACTA2) of mucosal fibroblasts by QPCR for characterizing fibroblast activation and collagen synthesis. The results showed that mucosal fibroblast activation can be inhibited using Pearl as a target.

In order to detect the effects of targeting Pearl in inhibiting adhesion formation *in vivo,* the inventors constructed an adhesion model in humanized Pearl animals and administered Pearl agonists to the wounded site at the same time. The results showed that Pearl agonists inhibited the ability of extracellular matrix synthesis by fibroblasts.

After knowing the function and mechanism of Pearl, based on this feature, Pearl can be used as a target to screening substances that promote the expression or activity of Pearl.

The present disclosure provides a method of screening substances (including potential substances) for preventing, alleviating or treating adhesive diseases, wherein the method comprises: contacting the candidate substance with a system that expresses Pearl or exists Pearl; and detecting the effect of candidate substance on Pearl; if the expression or activity of Pearl is elevated statistically by the candidate substance, then it indicates that the candidate substance is a substance used for preventing, alleviating or treating adhesive diseases.

In a preferred embodiment of the present disclosure, when performing screening, a control group may be established to facilitate the observation of changes in the expression or activity of Pearl and the signaling pathway proteins involved, or upstream and downstream proteins thereof. The control group can be a system expressing Pearl or containing Pearl without the addition of the candidate substance.

The expression system may be, for example, a cell (or cell culture) system, and the cells may be cells that express Pearl endogenously; or cells that express Pearl recombinantly. The system for expressing Pearl can also be (but not limited to) a subcellular system, a solution system, a tissue system, an organ system, an animal system (such as an animal model), and so on.

As a preferred embodiment of the present disclosure, the method also comprises: further cell experiments and/or animal experiments on the obtained substances (potential substances), so as to further select and determine substances really useful for preventing, alleviating or treating adhesive diseases.

The method for detecting the expression, activity, amount or secretion of Pearl is not particularly limited in the present disclosure. Conventional protein quantitative or semiquantitative detecting methods can be used, such as (but not limited to): SDS-PAGE method, Western-Blot method, ELISA and so on.

On the other hand, the present disclosure also provided compounds, compositions or drugs, or some potential substances obtained by the screening method. Some initially screened substances can constitute a screening library, so that people can finally screen out substances that are really useful for preventing, alleviating or treating adhesive diseases and useful in clinical.

### Pearl agonists and compositions

In the present disclosure, "agonist" comprises up-regulators, acting by promoting the expression of Pearl gene. When more Pearl protein is expressed (multiply expressed), it usually means the activity of Pearl increased, thereby resulting in inhibiting adhesive diseases.

In the present disclosure, any substance that can increase the activity of Pearl, increase the stability of Pearl, up-regulate the expression of Pearl, promote the secretion of Pearl and increase the effective time of Pearl can be used in the present disclosure as useful substances for up-regulating Pearl. All of these can be called as "agonists" or "up-regulators". The up-regulator can be an up-regulator in activities or expressions.

As used herein, the terms "agonist", "Pearl agonist" or "agonist of Pearl gene or a protein thereof" may be used interchangeably to refer to substances capable of enhancing the activity of Pearl gene or protein thereof, and/or binding Pearl protein, and/or promoting Pearl to inhibit signaling pathways of fibroblast activationsignaling pathway, and/or reducing ECM released from fibroblasts. In a specific embodiment, the present disclosure provides a Pearl agonistic antibody using Pearl as a regulatory target with a good inhibitory effect on adhesive diseases.

As an embodiment of the present disclosure, the Pearl up-regulator comprises (but not limited to): an expression vector or an expression construct capable of expressing (preferably over-expressing) Pearl after being transferred into cells. Usually, the expression vector comprises a gene cassette, and the gene cassette comprises the gene encoding Pearl and the regulatory sequences operatively linked thereto. The term "operably linked" or "operably linked to" refers to a condition that certain parts of a linear DNA sequence can regulate or control the activity of other parts of the same linear DNA sequence. For example, a promoter is operably linked to a coding sequence if it controls the transcription of the sequence.

In the present disclosure, the polynucleotide sequence of Pearl can be inserted into a recombinant expression vector, so that it can be transferred into cells and over-expressed to produce Pearl. Any plasmid and vector can be used as long as it can replicate and be stable in the hosts. An important characteristic of an expression vector is that it usually contains an origin of replication, a promoter, a marker gene, and translation control elements. For example, the expression vectors comprise: viral vectors, non-viral vectors and so on.

Preferably, the agonist provided by the present disclosure is an agonist which is capable of binding to a Pearl gene or a protein thereof and resulting a structure change of Pearl, thereby inhibiting fibroblast activation. Preferably, the agonist is an agonist targeting and binding to Pearl protein.

In a preferred embodiment, in addition to the antibody of the present disclosure, the agonist of the present disclosure may further comprise a substance that has a function of activating Pearl, such as FcεR1α (i.e., Fc fragment of IgE receptor Ia), dextran sulfate, fucose, or a combination thereof, and it may also comprise natural aptamer to Pearl and/or small molecule compound, more preferably, FcεR1α, dextran sulfate and fucose, with abilities of significantly inhibiting ECM synthesized by lung fibroblasts, improving lung function and increasing survival rate.

By using Pearl as a target of the present disclosure, a person skilled in the art would be able to design a variety of different agonists targeting Pearl, including, but not limited to, small molecule agonists, antibodies or active fragments thereof, miRNAs, and so on. In the present disclosure, the small molecule compound may also refer to a naturally occurred or artificially developed small molecule agonist to Pearl receptor.

In the present disclosure, the aptamer (Aptamer) refers to an in vitro screening technology: systematic evolution of ligands by exponential enrichment (Systematic evolution of ligands by exponential enrichment, SELEX). The obtained structured oligonucleotide sequence (RNA or DNA) has high recognition capability and high affinity with corresponding target molecules (proteins, viruses, bacteria, cells, heavy metal ions and so on).

As a preferred embodiment of the present disclosure, an antibody or variant thereof that specifically binds to/targets the Pearl protein is used, and the antibody or variant thereof has been confirmed to have a definite activation of Pearl at the level of cytology and zoology. Preferably, the antibodies or variants thereof of the disclosure are monoclonal antibodies derived from mice, and could be recombinant, polyclonal, multi-chained or single-chained, or intact immunoglobulin. Preferably, the antibodies or variants thereof in the present disclosure are humanized. Preferably, the antibodies of the present disclosure are chimeric antibodies. Preferably, the antibodies or variants thereof in the present disclosure are shown in Tables 1 to 4.

The antibody targeting Pearl comprises a full-length antibody and an antibody fragment (such as Fab', Fab', F(ab')₂, Fv such as scFv), and also comprise a bispecific antibody, a multispecific antibody, a single domain antibody or a monoclonal antibody or polyclonal antibody prepared from the antibody. The Fab fragment of the antibody is also called a fragment of antigen binding (Fragment of antigen binding), and refers to the domain in an antibody structure that binds to an antigen. Fab fragment consists of a complete light chain and VH and CH1 domain of a heavy chain. Under the action of papain, the antibody can be degraded into two Fab fragments and a Fc fragment. Under the action of papain, the antibody can be degraded into a (Fab)2 fragment and a Fc fragment.

FR fragments of Fab which can be used in the antibody or the variant thereof in the present disclosure are not particularly limited, preferably, FR of human IgG4 and many mutated FR sequences can be used in the present disclosure. The result shows that changing in FR sequence barely affects the binding between the antigen and the antibody. It could be seen that the CDR fragments of the antibody of the present disclosure has an excellent ability to specifically and stably bind to Pearl protein. A person skilled in the art would have been able to use the antibodies or variants thereof in the present disclosure according to the teaching of Pearl as a target of the present disclosure and the relevance to fibrotic diseases thereof, and use a plurality of currently known FR to screen the constructed antibodies, thereby obtaining an antibody having consistent or substantially consistent activity with the antibodies or the variants thereof in the present disclosure. It may also be included in the scope of the present disclosure.

Constant regions that can be used in the antibodies or variants thereof of the present disclosure are not particularly limited. A person skilled in the art would have been able to screen a constant region fragment suitable for an antibody containing the antibody or Ta variant thereof according to the teaching of Pearl as a target of the present disclosure and the relevance to fibrotic diseases thereof, thereby obtaining a complete antibody having consistent or substantially consistent activity with the antibodies or variants of the present disclosure.

The present disclosure provides a composition comprising effective amount (for example 0.000001-20wt%; preferably 0.00001-10wt%) of the Pearl or an agonist thereof (such as an agonist or an expression vector that overexpresses the Pearl) or an analog thereof, and a pharmaceutically acceptable carrier.

The composition of the present disclosure can be directly used for preventing, alleviating or treating adhesive diseases, and can also be used in combination with other therapeutic agents or auxiliary agents.

Generally, these materials can be formulated in a non-toxic, inert medium with pharmaceutically acceptable aqueous carrier, wherein the pH is usually about 5-8, preferably the pH is about 6-8.

The composition of the present disclosure comprises a safe and effective amount of Pearl, or an agonist thereof (such as an expression vector that overexpresses the Pearl), or an analog thereof, and a pharmaceutically acceptable carrier. Such excipients include (but are not limited to): saline, buffer, glucose, water, glycerol, ethanol, and combinations thereof. Usually, the pharmaceutical formulation should match the route of administration. The pharmaceutical composition of the present disclosure can be prepared in the form of an injection, for example, using a saline solution or a water solution containing glucose and other excipients through conventional methods. The pharmaceutical composition should be manufactured under sterile conditions. The amount of active ingredient administered is a therapeutically effective amount. The pharmaceutical formulations of the present disclosure can also be prepared into sustainedrelease formulations.

The effective amount of Pearl, or an agonist thereof in the present disclosure may vary with the route of administration, the severity of the disease to be treated, and the like. Selection of preferred effective amount can be determined by those skilled in the art based on various factors (e.g. through clinical trials). Such factors comprise but are not limited to: pharmacokinetic parameters (such as bioavailability, metabolism, half-life, and so on) of the Pearl or agonist thereof, the severity of the disease to be treated, weight, immune status of patients, the route of administration, and so on.

The present disclosure also provides a method for preventing, alleviating or treating adhesive diseases, comprising administering to a subject an effective amount of Pearl or an agonist thereof (such as an agonistic antibody or an expression vector overexpressing Pearl).

The route of administrating Pearl or its agonist of the present disclosure is not particularly limited. It may be systemic or local. For example, Pearl or the agonist thereof in the present disclosure can be but limited to: injectable formulations (such as intracavitary (non-venous), intravenous, intramuscular, subcutaneous, intradermal, and acupoint injections), transdermal formulations (such as lotions, coatings, ointments, transdermal plasters, etc.), mucosal formulations (such as oral films, sublingual tablets, mouthwashes), gastrointestinal formulations (such as decoctions, mixtures, granules, pills, tablets, etc.), inhalation formulations for respiratory delivery (such as aerosols, dry powder inhalers, aerosol inhalers), rectal formulations (such as suppositories, enemas), or combinations thereof.

As an embodiment of the present disclosure, the Pearl can be directly administered to mammals (such as humans), or the gene encoding Pearl can be cloned into an appropriate vector (such as conventional prokaryotic or eukaryotic expression vectors, or virus vectors such as herpes virus vectors or adenovirus vectors) by conventional methods, then the vectors can be introduced into cells that can express the Pearl, so that the cells express Pearl. Pearl expression can be achieved by introducing an appropriate amount of the cells into an appropriate part in a mammal.

The route of administrating Pearl or an agonist thereof mainly depends on the type and properties of the agonist.

The disclosure if further illustrated by the specific examples described below. It should be understood that these examples are merely illustrative, and do not limit the scope of the present disclosure. The experimental methods without specifying the specific conditions in the following examples generally used the conventional conditions, such as those described in J. Sambrook, Molecular Cloning: A Laboratory Manual (3^{rd} ed. Science Press, 2002) or followed the manufacturer's recommendation.

### Experimental materials and methods

### 1. Antibody Preparation

Anti-human Pearl monoclonal antibodies were prepared by the present inventors by using human Pearl extracellular protein to immunize BALB/c mice. High-affinity monoclonal hybridoma cells were prepared through standard mouse spleen cells and mouse myeloma cell fusion technology, and then obtained by standard antibody sequencing, 293S cell expression, and purification.

The light chain variable region and heavy chain variable region sequences of anti-human Pearl monoclonal antibodies LF2, LF3, LF7, LF11, LF15,and LF16 are listed in Table 1, and partially their CDR sequences are shown in Table 2.

**Table 1**

| Antibody | heavy chain variable region | light chain variable region |
|---|---|---|
| | (VH) sequence | (VL) sequence |
| LF2 | SEQ ID NO: 1 | SEQ ID NO: 16 |
| LF3 | SEQ ID NO: 2 | SEQ ID NO: 16 |
| LF7 | SEQ ID NO: 3 | SEQ ID NO: 17 |
| LF11 | SEQ ID NO: 2 | SEQ ID NO: 18 |
| LF15 | SEQ ID NO: 4 | SEQ ID NO: 19 |
| LF16 | SEQ ID NO: 5 | SEQ ID NO: 20 |

**Table 2**

| Antibody | SEQ ID NO: (HCDR1) | SEQ ID NO: (HCDR2) | SEQ ID NO: (HCDR3) |
|---|---|---|---|
| LF2 | 26 | 27 | 28 |
| LF3 | 26 | 27 | 29 |
| LF7 | 26 | 30 | 28 |
| LF11 | 26 | 27 | 29 |
| LF15 | 31 | 27 | 32 |
| LF16 | 33 | 34 | 35 |

| Antibody | SEQ ID NO: (LCDR1) | SEQ ID NO: (LCDR2) | SEQ ID NO: (LCDR3) |
|---|---|---|---|
| LF2 | 45 | GAT | 46 |
| LF3 | 45 | GAT | 46 |
| LF7 | 47 | SAS | 48 |
| LF11 | 49 | DTS | 50 |
| LF15 | 51 | GAT | 52 |
| LF16 | 45 | GAT | 46 |

The present disclosure also adopts the method of CDR transplantation to carry out humanization on the above-mentioned antibodies and carry out different back mutations to the sites of the framework region. The sequences of humanized antibodies hLF101-116, hLF-N55S, hLF-N55D, hLF-N55Q, hLF-G56A and hLF-G56V are also shown in Table 3.

**Table 3. Sequences of the antibodies**

| Antibody | heavy chain variable region (VH) sequence | light chain variable region (VL) sequence |
|---|---|---|
| hLF101 | SEQ ID NO: 7 | SEQ ID NO: 22 |
| hLF102 | SEQ ID NO: 7 | SEQ ID NO: 23 |
| hLF103 | SEQ ID NO: 7 | SEQ ID NO: 24 |
| hLF104 | SEQ ID NO: 7 | SEQ ID NO: 25 |
| hLF105 | SEQ ID NO: 8 | SEQ ID NO: 22 |
| hLF106 | SEQ ID NO: 8 | SEQ ID NO: 23 |
| hLF107 | SEQ ID NO: 8 | SEQ ID NO: 24 |
| hLF108 | SEQ ID NO: 8 | SEQ ID NO: 25 |
| hLF109 | SEQ ID NO: 9 | SEQ ID NO: 22 |
| hLF110 | SEQ ID NO: 9 | SEQ ID NO: 23 |
| hLF111 | SEQ ID NO: 9 | SEQ ID NO: 24 |
| hLF112 | SEQ ID NO: 9 | SEQ ID NO: 25 |
| hLF113 | SEQ ID NO: 10 | SEQ ID NO: 22 |
| hLF114 | SEQ ID NO: 10 | SEQ ID NO: 23 |
| hLF115 | SEQ ID NO: 10 | SEQ ID NO: 24 |
| hLF116 | SEQ ID NO: 10 | SEQ ID NO: 25 |
| hLF-N55S | SEQ ID NO: 11 | SEQ ID NO: 22 |
| hLF-N55D | SEQ ID NO: 12 | SEQ ID NO: 22 |
| hLF-N55Q | SEQ ID NO: 13 | SEQ ID NO: 22 |
| hLF-G56A | SEQ ID NO: 14 | SEQ ID NO: 22 |
| hLF-G56V | SEQ ID NO: 15 | SEQ ID NO: 22 |

The CDR sequences of the antibodies of the present disclosure are shown as SEQ ID NO: 26-55 in Table 4 below, and the CDR sequences of these antibodies adopt the IMGT numbering rules.

**Table 4. CDR sequences under the IMGT numbering rules**

| Antibody | SEQ ID NO(HCDR1) | SEQ ID NO(HCDR2) | SEQ ID NO(HCDR3) |
|---|---|---|---|
| LF2 | 26 | 27 | 28 |
| hLF101-106 | 39 | 27 | 28 |
| hLF-N55S | 39 | 40 | 28 |
| hLF-N55D | 39 | 41 | 28 |
| hLF-N55Q | 39 | 42 | 28 |
| hLF-G56A | 39 | 43 | 28 |
| hLF-G56V | 39 | 44 | 28 |

| Antibody | SEQ ID NO(LCDR1) | SEQ (LCDR2) | SEQ ID NO(LCDR3) |
|---|---|---|---|
| LF2 | 45 | GAT | 46 |
| hLF101-116 | 45 | GAT | 46 |
| hLF-N55S | 45 | GAT | 46 |
| hLF-N55D | 45 | GAT | 46 |
| hLF-N55Q | 45 | GAT | 46 |
| hLF-G56A | 45 | GAT | 46 |
| hLF-G56V | 45 | GAT | 46 |

### 2. Model Construction

### 2.1 construction of Mucosal adhesion model: peritoneal adhesion animal model

The model was made in humanized mice within 3 cm from the caecums of vermiform in peritoneal cavity, following the methods in the literature (Zhiliang Zhang, et al., Characteristics of genesis and development of peritoneal adhesion by different causes: experiment with rats, Chinese Journal of Medicine, 2005). The mice were divided into 3 groups. groups, with 20 mice in each group: A, control group with normal saline; B, prophylactic intraperitoneal injection of anti-human Pearl monoclonal antibody (including LF2 or hLF105 antibody) 0.5mg/kg, once a week; C, prophylactic intraperitoneal injection of anti-human Pearl monoclonal antibody 1mg/kg, once a week. After the mice were euthanized at 2 time points of 2 weeks and 4 weeks, an inverted "U"-shaped incision was selected to enter the abdomen, avoiding the original surgical incision. The entire abdominal cavity was observed for adhesion conditions, and samples of adhesive tissues and lavage fluid were collected for further observation and testing. And the degree of adhesion in the peritoneal adhesion model was evaluated .

### 2.2 Construction of subcutaneous mucosal adhesion model

Mice were anesthetized by intraperitoneal injection of pentobarbital sodium. The hair is depilated and sterilized, and the skin is excised. A thin layer of bonding agent was applied to one side of the silicone plate to fix the wound concentrically with a clamping board. Each clamping board was sutured at regular intervals with eight stitches to secure it to the intact tissue around the wound, preventing contraction of the wound edges. Sterile dressings were cut into circular shapes and attached to the silicone board for protecting the wound, preventing the animals from scratching or chewing. The anti-human Pearl monoclonal antibody was dripped prophylactically into the abdominal cavity in the peritoneal adhesion model.

### 3. Model maintenance and photography

Mice were anesthetized using isoflurane, maintaining a flow of 2% isoflurane. The sterile dressings on the silicone board were peeled off (or not peeled off). Pictures were taken at the wounded sites next to the fixed position and scale bar. If necessary, sterile dressings can be reapplied.. Image J was used to process the photos. The wounded area can be calculated according to the circled area by the scale.

### 4. Hematoxylin and Eosin (HE) Staining and Masson Staining

Mouse lung and liver were fixed in 4% paraformaldehyde for 48 hours, after paraffin embedding, a semi-automatic rotary slicing machine (Leica; rm2235 and cm1950) was used for slicing, dewaxing is carried out at room temperature, gradient ethanol is used for rehydration, HE and Masson staining were then performed.

### 5. Detection of hydroxyproline

After the treatment of starvation in cultured mucosal or subcutaneous derived fibroblasts for 12 hours, 1 µg/mL anti-human Pearl monoclonal antibody (including LF2 antibody or hLF105 antibody) was added separately to treat the cells. After 30 minutes, 10ng/mL TGFβ was added to treat the cells for 48 hours, then the cell supernatant was collected. The content of hydroxyproline was detected using the hydroxyproline detection kit of Nanjing Jiancheng Biology Co., Ltd. according to the instructions.

### 6. Quantitative PCR (QPCR)

Mucosal fibroblasts in Method 5 were collected. Total mRNA was extracted by Trizol method (Invitrogen, Carlsbad, CA) according to standard protocols. Complementary DNA was synthesized using reverse transcriptase (Takara). ABI7000 Real-time PCR instrument (Life Technologies) was used to detect gene expression and the PCR primer sequences are shown in Table 5. The 18sRNA gene was used as an internal reference.

**Table 5. qPCR primer sequences**

| Gene | Forward primer | Reverse primer |
|---|---|---|
| Col 7a1 | caggcattggtgccagtgaaca(SEQ ID NO: 104) | tggacctccatcctcacagtca(SEQ ID NO: 105) |
| ACTA2 | tgctgacagaggcaccactgaa(SEQ ID NO: 106) | cagttgtacgtccagaggcatag(SEQ ID NO: 107) |
| 18sRNA | ttgactcaacacgggaaacc(SEQ ID NO: 108) | agacaaatcgctccaccaac(SEQ ID NO: 109) |

### 7. Construction of Pearl humanized transgenic mice

The human Pearl gene was introduced into mice by CRISPR/Cas9 technology. Target sites were designed near exon 1 and the ATG start codon of the mouse Pearl gene. With the presence of a homologous recombinant template, the exogenous human Pearl gene was precisely integrated into the designated location, replacing the mouse Pearl gene sequence.

### 8. Statistical methods

All data were statistically analyzed using Graphpad8.0 software. The t test was used to compare the data between the two groups. Kaplan-Meier analysis was used for survival analysis. In all data comparisons, p-values less than 0.033 were considered statistically significant.

### Example 1. Correlation between subcutaneous mucosal fibroblasts and dermis adhesion

Fibroblast-specific Pearl knockout mice were used to observe the formation of subcutaneous mucosal adhesions and compare to the control group.

The results showed that Pearl-deficient mice had more severe subcutaneous mucosal adhesions than the control mice, with increased mucosal thickness at the wounded site, massive proliferation of fibroblasts, increased activated myofibroblasts, and increased area of collagen , as shown in Figure 1A -B.

This result showed that Pearl inhibited excessive activation of mucosal fibroblasts and the formation of subcutaneous mucosal adhesions. Therefore, Pearl could be used as a potential target for inhibiting mucosal adhesions.

### Example 2. The activation of mucosal fibroblasts can be inhibited using Pearl as a target.

To further investigate the role of targeting Pearl in inhibiting mucosal fibroblast activation, subcutaneous mucosal fibroblasts were isolated from humanized Pearl mice and cultured in vitro. TGFβ activated fibroblasts were treated with anti-human Pearl monoclonal antibodies (including LF2 or hLF105) for 48 hours, and the supernatants were collected to detect the content of hydroxyproline. The cells were collected for QPCR to detect the expression levels of extracellular matrix proteins such as collagen 7a1 (Col 7a1) and smooth muscle actin α2 (ACTA2) to characterize fibroblast activation and collagen synthesis.

The experimental results showed that, compared with the control group, anti-Pearl monoclonal antibodies (including LF2 or hLF105) inhibited the activation of fibroblasts and the ability to synthesize extracellular matrix, as shown in Figure 2A, B.

The experimental data showed that the activation of mucosal fibroblasts can be inhibited using anti-Pearl antibody with Pearl as a target. Besides, at early stages after injury, the antibody of this target used in mucosa can play a good role in preventing adhesions.

### Example 3. Anti-Pearl monoclonal antibody inhibits the formation of adhesions in the subcutaneous mucosal adhesion model of humanized Pearl mice

Subsequently, in order to detect the role of targeting Pearl in inhibiting adhesion formation in vivo, the inventors constructed an adhesion model in humanized Pearl mice, and at the same time instilled anti-human Pearl monoclonal antibodies (including LF2 or hLF105) each at a dose of 5 micrograms at the wounded site, with once administration every 7 days. On the 14th day, the subcutaneous mucosal tissue at the wounded site was removed, stained with HE and Masson, and photographed under a microscope. The thickness of the subcutaneous mucosa and skin and the area of collagen were counted by Image J software for indicating the formation of adhesions.

The results showed that anti-Pearl monoclonal antibody significantly reduced the thickness of dermal mucosa at the wounded site, the proliferation, hyperactivation and collagen synthesis of fibroblasts, as shown in Figure 3A, B, C.

These data all indicate that anti-Pearl monoclonal antibody can prevent and inhibit the formation of adhesions.

### Example 4. Anti-Pearl monoclonal antibody inhibits the formation of adhesions in the peritoneal mucosal adhesion model of humanized Pearl mice

For fibroblast-specific Pearl knockout mice, a peritoneal adhesion model was used to form a sham operation on the mice. The model preparation and animal grouping were as described above.

### 1. BhatiaDS score

According to the scoring standard of BhatiaDS, grade 0: no adhesion; grade I: adhesive area of the area treated in the caecums of vermiform/total area of the treated area< 50% , easy for blunt separation; grade II: above-mentioned area ratio ≥ 50%, easy for blunt dissection; Grade III: Regardless of the size of the adhesive area, as long as the adhesion is difficult to separate, blunt separation can still be performed, but the intestinal wall is obviously damaged after separation; Grade IV: Regardless of the size of the adhesive area, as long as the adhesion is firm and can no longer be bluntly separated, required sharp separation. Each mouse was scored by 3 people respectively, and the average value was taken as the score of adhesion in this sample.

The results showed that the BhatiaDS score of mice treated with anti-human Pearl monoclonal antibody (including LF2 or hLF105) was significantly lower than that of the control group (Table 6), especially in the high-dose group (1.0mg/kg). It is indicated that the use of Pearl antibodies can effectively prevent abdominal adhesions.

**Table 6**

| | LF2 | | hLF105 | | hLF107 | | hLF110 | | hLF-N55Q | |
|---|---|---|---|---|---|---|---|---|---|---|
| | 0.5 mg/kg | 1.0 mg/kg | 0.5 mg/kg | 1.0 mg/kg | 0.5 mg/kg | 1.0 mg/kg | 0.5 mg/kg | 1.0 mg/kg | 0.5 mg/kg | 1.0 mg/kg |
| Control | III-IV | IV | III-IV | IV | III-IV | IV | III-IV | IV | III-IV | IV |
| 2 weeks (n=8) | I | III | II | III | II | IV | I | III | II | III |
| 4 weeks (n=8) | 0-I | I | I | II | I | II | 0 | I | I | I |

### 2. Zuhlke Score

Tissue sections were stained by different staining methods. The thickness of the adhesive tissues, the degree of inflammatory cell infiltration, the proliferation of new capillaries, the proliferation of fibroblasts and interstitial cells, and the arrangement of collagen fibers were observed under a light microscope. The Zuhlke scoring standard is quantitative scoring: grade 1, Loose connective tissue with abundant cells. Mature and immature fibrin and reticular fibers can be seen; grade 2, Connective tissue containing cells and capillaries. A small amount of collagen fibers can be seen; grade 3, Firm connective tissue with few cells, numerous blood vessels, few elastic fibers and smooth muscle fibers; grade 4, Mature granulation tissue with few cells. The serosa layer is difficult to distinguish.

The results showed that the peritoneal adhesive tissue of mice treated with anti-human Pearl monoclonal antibody (including LF2 or hLF105, 1.0 mg/kg) was relatively loose and rich in cells, and the scores were all grade 1-2, while the control group had It is grade 4, all of which are dense connective tissue and granulation tissue. It can be seen that the use of Pearl antibody can effectively prevent abdominal adhesions. The results indicated that in mice treated with anti-human Pearl monoclonal antibodies (including LF2 or hLF105, 1.0mg/kg), the abdominal adhesive tissue was loose and abundant cells, with scores ranging from grade 1 to grade 2. In contrast, the control group exhibited dense connective tissue and granulation tissue, with all scores being grade 4. This suggested that the use of Pearl antibodies can effectively prevent abdominal adhesions.

### 3. Determination of hydroxyproline in tissues

The formation of tissue adhesion depends on the synthesis of collagen and hydroxyproline is the precursor of collagen synthesis. Based on this theory, the determination of hydroxyproline content in tissues can indirectly reflect the severity of adhesion, and the two are linearly positively correlated. Therefore, the determination of hydroxyproline level in the adhesive tissue was taken as another important method for adhesion assessment.

The results showed that the hydroxyproline content in tissues of mice treated with anti-human Pearl monoclonal antibody (including LF2 or hLF105) was significantly lower than that of the control group.

### 4. Determination of indicators related to adhesion in mice

Immunohistochemistry or RT-PCR was used to measure some indicators related to adhesion formation to reflect the severity of adhesion, such as: fibrinogen (FIB), α-smooth actin (α-SMA), MMPs/TIMPs, transforming growth factor (TGF-β1), vascular endothelial growth factor (VEGF).

The results showed that the indicators related to adhesion of mice treated with anti-human Pearl monoclonal antibody (including LF2 or hLF105) was significantly lower than that of the control group.

Each reference provided herein is incorporated by reference to the same extent as if each reference was individually incorporated by reference. In addition, it should be understood that based on the above teaching content of the disclosure, those skilled in the art can practice various changes or modifications to the disclosure, and these equivalent forms also fall within the scope of the appended claims.

## Claims

1. A use of Pearl gene, an encoded protein or an agonist thereof in the manufacture of pharmaceutical compositions for preventing, alleviating or treating adhesive diseases.

2. The use according to claim 1, wherein, the adhesive diseases comprise: mucosal adhesive diseases or serosal adhesive diseases; preferably, the adhesive diseases comprise adhesive diseases formed after injuries, bleeding, infections, foreign body stimuli, tumor infiltration, or inflammatory cell infiltration; preferably, the adhesive diseases formed after injuries comprise postoperative adhesions, adhesions after radiotherapy or chemotherapy; the adhesive diseases formed after infections comprise inflammatory adhesions; preferably, the adhesive diseases comprise adhesive diseases in digestive system, urinary system, respiratory system, reproductive system, oral cavity, joints and the inner layers of the skin; more preferably comprising adhesive diseases in abdominal cavity, pelvic cavity, thoracic cavity, uterus, joint cavities, pericardium, epidural space or periodontal sites; more preferably, the adhesive diseases are abdominal adhesions, pelvic adhesions, thoracic adhesions or joint cavity adhesions.

3. The use according to claim 1, wherein, the agonist of Pearl comprises agents that increase the expression, activity, stability, and/or effective time of Pearl; preferably, it comprises: an antibody that specifically activates Pearl, an antigen-binding fragment or a variant thereof, an expression construct for recombinantly expressing Pearl, a chemical agonist of Pearl, an up-regulator that promotes the driving ability by a promoter of Pearl gene; more preferably, the chemical agonist of Pearl comprise: FcεR1α, dextran sulfate, fucoidan, or combinations thereof.

4. The use according to claim 1, wherein, the Pearl gene, encoded protein or agonist thereof is also used to:
inhibiting mucosal fibroblast activation;
reducing the content of hydroxyproline in tissues;
reducing the thickness of the mucosa at the lesion site;
inhibiting fibroblast proliferation or activation at the lesion site;
decreasing the synthesis of collagen at the lesion site; or
down-regulating adhesion-related indicators, preferably the indicators comprise: fibrinogen, α-smooth actin, t-PA/PAI, MMPs/TIMPs, transforming growth factor, vascular endothelial growth factor, tumor necrosis factor and/or interleukin.

5. A method of preventing, alleviating or treating adhesive diseases, comprising administering the Pearl gene, encoded protein or agonist thereof to a subject in need of prevention, alleviation or treatment.

6. A pharmaceutical composition or drug kit for preventing, alleviating or treating adhesive diseases, wherein it comprises an effective amount of Pearl gene, encoded protein or agonist thereof, and a pharmaceutically acceptable carrier.

7. The use according to claim 1, the method according to claim 5 or the pharmaceutical composition according to claim 6, wherein, the agonist is an antibody specifically activates Pearl, an antigen-binding fragment or a variant thereof; preferably, the antibody comprises a heavy chain variable region and/or a light chain variable region, and the HCDR1, HCDR2 and HCDR3 amino acid sequences of the heavy chain variable region are as shown in SEQ ID NO: 26, 27 and 28, or as shown in SEQ ID NO: 26, 27 and 29, or as shown in SEQ ID NO: 26, 30 and 28, or as shown in SEQ ID NO: 31, 27 and 32, or as shown in SEQ ID NO: 33, 34 and 35; and/or the LCDR1, LCDR2 and LCDR3 amino acid sequences of the light chain variable region are as shown in SEQ ID NO: 45, GAT and SEQ ID NO: 46, or as shown in SEQ ID NO: 47, SAS and SEQ ID NO: 28, or as shown in SEQ ID NO: 49, DTS and SEQ ID NO: 50, or as shown in SEQ ID NO: 51, GAT and SEQ ID NO: 52;
more preferably, the heavy chain variable region of the antibody is as shown in SEQ ID NO: 1, 2, 3, 4, 5, 7, 8, 9, 10, 11, 12, 13, 14 or 15, or the light chain variable region of the antibody is as shown in SEQ ID NO: 16, 17, 18, 19, 20, 22, 23, 24 or 25.

8. A use of Pearl gene, an encoded protein thereof for screening drugs or compounds for preventing, alleviating or treating adhesive diseases.

9. A method of screening drugs or compounds for preventing, alleviating or treating adhesive diseases, comprising:
(1) treating an expression system by a candidate substance, wherein the system expressing Pearl or comprising Pearl; and
(2) detecting the system to observe the expression or activity of Pearl; if the expression or activity of Pearl is elevated statistically by the candidate substance, then it indicates that the candidate substance is the desired drug or compound.

10. The method according to claim 9, wherein, the step (1) comprises: in the testing group, adding candidate substances to the expression system; and/or,
the step (2) comprises: detecting the system to observe the expression or activity of Pearl and compared to the control group, wherein the control group is an expression system without adding the candidate substances; if the expression or activity of Pearl is elevated statistically by the candidate substance, then it indicates that the candidate substance is the desired drug or compound.
